(19) Europäisches Patentamt
European Patent Office
Office européen des brevets

(11) Publication number: **0 361 722 B1**

(12) **EUROPEAN PATENT SPECIFICATION**

(45) Date of publication of patent specification: **08.12.93**

(51) Int. Cl.5: **C09J 133/08**, A61L 15/58

(21) Application number: **89309194.2**

(22) Date of filing: **11.09.89**

(54) **Antibacterial adhesive composition.**

(30) Priority: **12.09.88 US 243389**

(43) Date of publication of application:
**04.04.90 Bulletin 90/14**

(45) Publication of the grant of the patent:
**08.12.93 Bulletin 93/49**

(84) Designated Contracting States:
**CH DE ES FR GB IT LI NL SE**

(56) References cited:
**EP-A- 0 272 149**
**EP-A- 0 297 769**
**US-A- 4 340 043**
**EP-A- 0 275 550**
**NL-A- 7 408 580**
**US-A- 4 551 490**

(73) Proprietor: **JOHNSON & JOHNSON MEDICAL, INC. (a New Jersey corp.)**
**2500 Arbrook Boulevard**
**Arlington Texas 76010(US)**

(72) Inventor: **Schonfeld, Edward**
**85 Dodds Lane**
**Princeton, NJ 08540(US)**

(74) Representative: **Fisher, Adrian John et al**
**CARPMAELS & RANSFORD**
**43 Bloomsbury Square**
**London WC1A 2RA (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to an antibacterial adhesive composition suitable for medical applications. More particularly, this invention relates to an effective antibacterial, acrylic-based, hydrophobic adhesive which includes silver sulfadiazine as the antibacterial agent and an internally crosslinked sodium carboxymethylcellulose as a release aid.

Various adhesive compositions suitable for medical application are known. Chen in U.S. Patent No. 3,339,545 discloses an adhesive composition comprising a blend of one or more water soluble or water swellable hydrocolloids and polyisobutylene. Kresner in U.S. Patent No. 4,393,150 discloses a bandage adhesive of polyisobutylene, polybutene, butyl rubber, reinforcing fiber, filler material and zinc oxide blended under heat and rolled into a thin sheet. Schonfeld in U.S. Patent No. 4,140,115 discloses adhesive bandage articles which minimize skin damage to the application area by providing a backing material, e.g., a plasticized film backing, having a water insoluble, pressure sensitive adhesive composition coated thereon which composition includes from about 4-20% by weight of an unreacted polyol uniformly dispersed therein.

For certain medical uses, e.g., burn dressings, it is often desirable to medicate the wound area with various medicaments including antibacterial agents. While antibacterial agents and other medicaments can be applied directly to the injured site, it is often convenient to apply medicament to the portion of the bandage that comes into contact with the injured area. In this manner, medicament is automatically and uniformly applied to the injured area whenever a bandage is applied thereto.

U.S. Patent No. 4,340,043 to Seymour discloses adhesive coated bandage sheet materials which incorporate up to 25% by weight of antibacterial substances in the adhesive coating to kill bacteria in a wound and surrounding covered skin area. Various antibacterial agents are disclosed including metal salts, antibiotics such as neomycin, antibacterials such as chlorhexidine and its salts, quaternary ammonium compounds, iodophors, and preferably silver sulfadiazine. Seymour teaches applying up to 25%, preferably up to 15%, by weight of silver sulfadiazine in finely divided form to adhesive compositions to provide antibacterial action. Seymour discloses that as low as 1% of silver sulfadiazine can be used but recognizes that for effective antibacterial properties a suitable range should start at about 5% silver sulfadiazine. The effective amount of silver sulfadiazine required by Seymour, i.e. 5% or more, is unsuitably high for reasons of safety and cost.

U.S. Patent No. 4,551,490 to Doyle et al. discloses pressure sensitive adhesive compositions suitable for various medical applications which are resistant to erosion by moisture and biological fluids. Doyle et al. disclose useful compositions as a homogenous blend of mineral oil, one or more polyisobutylenes or mixtures of one or more polyisobutylenes and an elastomer such as butyl rubber, styrene block or radial type copolymers, water-soluble hydrocolloid gums, water-swellable cohesive strengthening agents and a tackifier. Doyle et al. disclose the use of water-swellable adhesive strengthening agents to provide resistance to erosion by biological fluids of the adhesive compositions. Crosslinked sodium carboxymethycellulose, is identified as a suitable water-swellable cohesive strengthening agent. Doyle et al. also disclose that small amounts, i.e., less than 5% by weight of the adhesive composition may contain other optional ingredients, e.g., in burn dressings, active ingredients such as silver sulfadiazine and other silver compounds may be included. Doyle et al. do not discuss nor contemplate the effectiveness of silver sulfadiazine incorporated into adhesive and burn dressings.

While various adhesive compositions are known which may optionally include antibacterial agents such as silver sulfadiazine, improved adhesive compositions incorporating antibacterial agents such as silver sulfadiazine are needed which can provide more efficient and effective antibacterial action to a wound site. It is, therefore, an object of the present invention to provide silver sulfadiazine containing acrylic adhesive compositions which provide more efficient and effective antibacterial action, with smaller amounts of the active ingredient than have heretofore been practical. Effective use of smaller amounts of silver sulfadiazine improves cost and safety factors while preserving the therapeutic value of the products produced thereby.

### Summary of the Invention

The foregoing object of providing more efficient and effective antibacterial activity has now been accomplished by improving the release of silver sulfadiazine from acrylic-based adhesives in accordance with the compositions and methods of this invention.

In accordance with the purposes of the invention, as embodied and fully described herein, the invention comprises an antibacterial pressure sensitive adhesive composition suitable for medicinal application to an infection prone skin area comprising a mixture of a solid hydrophobic acrylic adhesive; an antibacterial

effective amount of silver sulfadiazine; and an effective amount of internally crosslinked sodium carboxymethylcellulose to release the silver sulfadiazine from the adhesive mixture to provide effective antibacterial action to the area of application. In preferred embodiments of the invention, the adhesive compositions comprise by weight of the total compositions, from 40 to 50% of solid hydrophobic acrylic adhesive; 0.5 to 3.0% of silver sulfadiazine and 1 to 10%, more preferably 2 to 3% of internally crosslinked sodium carboxymethylcellulose.

The invention also comprises a method of reducing bacteria in an infection prone skin area comprising applying thereto an antibacterial pressure sensitive adhesive composition comprising a mixture of a hydrophobic adhesive; an antibacterial effective amount of silver sulfadiazine; and an amount of internally crosslinked sodium carboxymethylcellulose sufficient to make an antibacterial effective amount of the silver sulfadiazine available to the infection prone skin area to reduce the bacteria present in the area.

## Detailed Description of the Preferred Embodiments of the Invention

Reference will now be made in detail to present preferred embodiments of the invention, examples of which are illustrated in the following examples section. It is to be understood that both the foregoing general and the following detailed description are exemplary and explanatory only and are not intended to be restrictive of the invention as claimed.

The antibacterial pressure sensitive adhesive compositions of the invention are suitable for various medical applications, particularly, for use in wound and burn dressings. The pressure sensitive bandages and wound dressings of the invention are contemplated to be applied to wound or burn areas of the skin where the protective epidermal layer of the skin has been removed or damaged thus exposing internal layers of the skin to possible infection from outside bacterial and microbial sources. This area of the skin to which the bandage or dressing is to be applied is defined for purposes of the invention as the "infection prone skin area". Infection prone skin areas may also include areas subjected to chafing, skin disorders or skin degenerative diseases which also make the effected skin area prone to infection.

The pressure sensitive adhesive compositions of the invention may be prepared in the form of adhesive strips or bandages comprising backing materials including but not limited to plasticized film backings such as plasticized polyvinyl chloride, as well as, woven and nonwoven fabrics, paper and metallic foil bodies and foamed materials such as foamed polyurethane.

The present invention is not to be construed as relying upon any particular mechanism or mode of action for migration of the silver sulfadiazine active ingredient from the adhesive layer to the infection prone skin area. The present invention rests upon the discovery that inclusion of an internally crosslinked sodium carboxymethylcellulose in an acrylic-based adhesive vastly improves the antibacterial effectiveness of surprisingly small amounts of silver sulfadiazine incorporated therein. The internally crosslinked sodium carboxymethylcellulose promotes the effectiveness of the silver sulfadiazine by facilitating release or migration of silver sulfadiazine from the adhesive mixture and increasing its availability to the infection prone skin area. It is theorized that addition of internally crosslinked sodium carboxymethylcellulose transports moisture from the wound area and atmosphere to the adhesive matrix to facilitate its disintegration thereby releasing silver sulfadiazine thus dramatically increasing the antibacterial effectiveness of the combination.

The antibacterial adhesive compositions of the invention are particularly advantageous because only small amounts of silver sulfadiazine need to be present in the adhesive composition to provide effective antibacterial action to the infection prone skin area. Effective antibacterial action is defined as reduction of bacteria on the order of at least 100 (log 2.0) versus an unprotected area of the skin. The antibacterial adhesive compositions of the invention have demonstrated reduction of bacteria on an order greater than 200 (log 2.30) to over 300.

Preferred amounts of silver sulfadiazine are 0.5 to 3.0 percent by weight of the total adhesive composition, more preferably about 1.0 percent. Smaller amounts of silver sulfadiazine provide obvious cost and safety benefits to products incorporating the compositions and methods of the present invention.

Amounts of the internally crosslinked sodium carboxymethylcellulose which are effective to increase the antibacterial effectiveness of the silver sulfadiazine is at least one percent by weight, more preferably 2 to 3% of the total adhesive mixture exclusive of solvents and up to an amount that is practical to provide sufficient release qualities to the adhesive without deleteriously effecting the adhesive and cohesive properties of the compositions. A preferred upper limit would be about 10% by weight of the adhesive mixture comprising the adhesive solids and silver sulfadiazine.

The presently preferred internally crosslinked carboxymethylcellulose is sold under the brand name Ac-Di-Sol by FMC corporation. This internally crosslinked sodium carboxymethylcellulose, also called modified

cellulose gum, is a cellulose ether produced by reacting alkali cellulose with sodium monochloroacetate under prescribed reaction conditions to produce an internal or intrachain crosslink. Normal sodium carboxymethylcellulose is generally water-soluble but the internally crosslinked form is essentially water-insoluble. The internally crosslinked product is highly absorbent and is known as an effective disintegrant. Comparative testing of internally crosslinked sodium carboxymethylcellulose against other water soluble or water absorbent polymers and disintegrants reveals that internally crosslinked sodium carboxymethylcellulose is considerably more effective in promoting the antibacterial effectiveness of identical amounts of silver sulfadiazine within the adhesive mixtures. Results of this comparative testing is disclosed in the examples section, infra.

The preferred antibacterial agent in accordance with the invention is silver sulfadiazine in finely divided form. The size of the silver sulfadiazine particles are generally less than 20 microns in diameter and most are less than 5 microns in diameter and are imbedded in the adhesive layer mixture. Finely divided particles of less than 20 microns in diameter are preferred because the small size contributes to more effective dispersion and solubility of the silver sulfadiazine in the wound fluids.

Although adhesive solids based on rubber elastomers which include a polyol, are satisfactory bandage adhesives, the preferred adhesive masses in accordance with the invention are based on acrylic systems. Such acrylate masses consist essentially of a major amount of a medium chain length alkyl acrylate monomer and optionally minor amounts of cohesion-inducing short chain monomers, plus a very small amount of an alkoxy silyl crosslinking monomer polymerizable in the acrylate system.

Useful medium chain length alkyl acrylate monomers generally are those averaging 4-12 carbon atoms in the alcohol moiety and include: butyl, hexyl, 2-ethylhexyl, octyl, decyl, and dodecyl acrylates and the like, alone or in combination with one another or with higher and lower alkyl acrylates. The medium chain length acrylate monomer is present in the adhesive copolymer of this invention in a major amount by weight of the total monomers.

Cohesion-inducing short chain monomers are generally selected from vinyl acetate, methyl acrylate, acrylic acid, diacetoneacrylamide, N-t-butylacrylamide, and the like.

Alkoxy silyl crosslinking monomers comprises an alkoxy silyl alkyl group and an unsaturated functional terminal group copolymerizable with the other monomers. This functional terminal group is preferably an acrylate or substituted acrylate group such as:

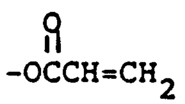 or 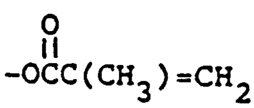

The polymerizable crosslinking alkoxy silyl alkyl groups found to be particularly effective are those having the general formula:

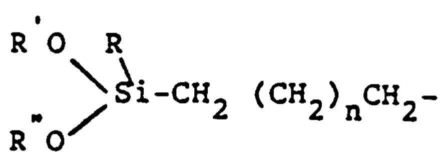

where R′ and R" are either $CH_3$ or $CH_3CH_2$ and R is selected from the group consisting of $CH_3$; $CH_2$; $CH_3O$; and $CH_3CH_2O$ and n is an integer of 0 through 8. A preferred silyl crosslinking monomer is 3-methacryloxypropyltrimethoxy silane.

The amount of the silyl crosslinking monomer to be included in the copolymer depends on the exact constituents of the copolymer and the degree of crosslinking desired. The silyl crosslinking monomer will generally be included in amounts of 0.005 to 0.1 or more parts by weight of the total monomers with 0.01 to 0.05 parts by weight being preferred. A fuller disclosure of acrylate polymers and other useful adhesive compounds such as addition of polyols to reduce skin irritation is described in U.S. Patent No. 4,140,115. The entire disclosure of this patent is hereby incorporated herein by reference to provide further teachings of examples of adhesive components of the invention.

In those adhesive mass compositions based on an acrylate resin, it is the usual practice to employ a solvent coating technique in order to apply the adhesive mass to the article or backing material to be coated. In well known conventional techniques, an organic solvent, such as cyclohexane or ethyl acetate, is

employed.

Suitable backing material can be a plasticized film backing of polyvinyl chloride, a copolymer of polyvinyl chloride and polyvinyl acetate, a copolymer of polyvinyl chloride and acrylonitrile and self-supporting film materials, as well as, cloth and paper wherein such paper can be used alone or as a carrier for a plasticized organosol.

It is, therefore, apparent that the compositions and methods of the invention are effective for promoting the antibacterial activity of silver sulfadiazine in adhesive mixtures, particularly acrylic adhesive mixtures, because of the presence of internally crosslinked carboxymethylcellulose in fulfillment of the objects of the invention.

## Examples

The invention will now be illustrated by examples. The examples are not intended to be limiting of the scope of the present invention. In conjunction with the detailed and general description above, the examples provide further understanding of the present invention and outline a process for producing compositions of the invention.

The following examples represent preferred embodiments of the compositions, processes and methods of the invention to satisfy the stated objects of the invention. The starting materials and reagents used in the examples whose method of preparation are not indicated are commercially available to sources known to the art such as chemical supply house.

### Example I

Preparation of an Adhesive Dressing Containing a Releasable Biological Active Ingredient

A typical formulation containing 1% silver sulfadiazine (SSD) by weight of total solids in a solvent based acrylic hydrophobic adhesive is shown below:

| | Gram Weight |
|---|---|
| Acrylic Adhesive 2674 (about 45% solids) | 985.56 |
| SSD | 4.58 |
| Internally crosslinked carboxymethylcellulose (Ac-Di-Sol brand from FMC). | 9.86 |
| | 1000.00 |

The acrylic adhesive is prepared as follows:

| Ingredients: | Parts by Weight |
|---|---|
| A. 2-Ethylhexyl Acrylate | 350.0 |
| B. Vinyl Acetate | 150.0 |
| C. Silane | 0.10 (0.09 ml) |
| D. Ethyl Acetate | 612.5 |
| E. Lauroyl Peroxide | 3.13 |

Charge Items A - D into a 2-liter flask. Heat to 72°C under a $N_2$ atmosphere. Turn off $N_2$, add 2.50 parts by weight of Item E and continue to heat until an exotherm occurs. Control the exotherm (82-84°C) by cooling the outside walls of the flask. At the conclusion of the exotherm, resume heating at reflux for 3 hours. Add an additional 0.63 parts by weight of Item E and continue to reflux for 2 hours. Cool and discharge.

The additives (SSD and carboxymethyl cellulose) are mixed together and then slowly added to a stirred solution of the adhesive. After 30 minutes of stirring, the dispersion is homogeneous and is coated onto release paper at a wet thickness of 5 mm and air dried. The adhesive film is transfer coated to polyurethane film. The adhesive film is uniform in appearance, light in color, and without any apparent undispersed

particles.

Comparative Examples II-IX

Eight comparative examples comprising water soluble or water absorbent polymers added to acrylic adhesive together with 1% SSD and coated onto polyurethane film are prepared in accordance with the procedures of Example I, as well as, examples using no polymeric additive and no SSD or polymeric additives.

Examples II - VII

The procedure for Example I is followed except that Ac-Di-Sol is replaced with the following polymer additives as indicated:

Example II

2.1% AVICEL brand water insoluble microcrystalline cellulose from FMC and 2.1% Ac-Di-Sol.

Example III

2.1% KLUCEL brand water soluble hydroxypropyl ether of cellulose from Hercules.

Example IV

2.1% NATROSOL brand water soluble hydroxyethyl ether of cellulose from Hercules.

Example V

2.1% CMC brand water insoluble sodium carboxymethyl ether of cellulose from Hercules.

Example VI

2.1% EXPLOTAB brand water insoluble sodium carboxymethyl ether of potato starch from Mendall and 2.1% SOLKA-FLOC brand water insoluble powdered cellulose from Mendall.

Example VII

2.1% METHOCEL brand water soluble hydroxypropyl ether of methylcellulose from Dow and 2.1% Ac-Di-Sol.

Comparative Examples VIII and IX

Example VIII is prepared as above except only acrylic adhesive and no SSD or polymer additive is used. Example IX includes the acrylic adhesive and SSD only.

In vitro testing of antibacterial effectiveness of Examples I-IX

Dressings of Examples I-IX containing a 1% level of SSD (no SSD was included in EX. VIII) are evaluated for their bactericidal activities against Ps. aeruginosa present in experimentally contaminated full thickness excisions on guinea pigs. Microbial sampling of the wound beds 24 hours post-treatment revealed the following mean log reduction of viable Ps. aeruginosa as compared to the untreated controls. (See Table 1 infra.)

METHOD

Pseudomonas aeruginosa is the test organism used in this experiment. Preparation of the organism for wound inoculation consisted of culturing in Trypticase Soy Broth for 24 hours at 35 °C. Collection of cells by centrifugation, washing with sterile saline, and restoring to original volume. The cells are then diluted 1-1000 and the latter dilution used for inoculation.

ANIMAL MODEL

Circular full-thickness mid-dorsal excisions approximately $4cm^2$ are surgically inflicted upon anesthetized and shaved adult male guinea pigs, weighing between 400 and 500 gms. The wounds are inoculated with 0.01 milliliters of Ps. aeruginosa suspension and the wounds treated with the designated dressing for each group, (secured with ELASTIKON brand adhesive tape from Johnson & Johnson). Following a 24 hour treatment period the wound beds are sampled by swabbing with cotton swabs. Each swab is placed into 10 milliliters of 1% Sodium Citrate and dispensed by vortexing. Serial dilutions were made and plated in Trypticase Soy Agar, and incubated for 48 hours at 35 °C. After 48 hours the plates are examined and the number of CFUs (colony forming units) enumerated.

RESULTS

The results obtained are shown in Table I below.

Table 1

| Ex. | Composition | Logarithmic Reduction of Ps. aeruginosa |
|---|---|---|
| I | SSD + Ac-Di-Sol | 2.49 |
| | Comparative Examples | |
| II | SSD + AVICEL + Ac-Di-Sol | 2.08 |
| III | SSD + KLUCEL LF | 2.03 |
| IV | SSD + NATROSOL 250L | 1.66 |
| V | SSD + CMC 7LXF | 1.06 |
| VI | SSD + EXPLOTAB + SOLKA-FLOC | 1.20 |
| VII | SSD + METHOCEL + Ac-Di-Sol | 1.50 |
| VIII | SSD | 0.77 |
| IX | No SSD | Control |

In summary, it was determined that only the adhesive additive of Example I consisting of only Ac-Di-Sol, an internally cross-linked modified cellulose gum from FMC and SSD, was highly effective in promoting the release of SSD from a hydrophobic acrylic adhesive, thereby resulting in a significantly increased reduction in the concentration of the microorganisms present.

The scope of the present invention is not limited by the description, examples and suggested uses herein and modifications can be made without departing from the spirit of the invention. For example, other additives may be included in the adhesive mixture of the invention such as analgesic compositions, other medicaments, or other additives which may increase the cohesive strength of the adhesive mixture or impart other desirable properties thereto.

Application of the compositions and methods of the invention and the products made thereby can be to various fields as would be presently and prospectively known and recognized by those skilled in the art. Application of the products of the invention to medical uses can be accomplished by any suitable therapeutic method and technique such as those known to those skilled in the art of wound and burn dressings. Thus, it is intended that the present invention cover the modifications and variations of this invention provided that they come within the scope of the appended claims and their equivalents.

## Claims

1. An antibacterial pressure sensitive adhesive composition suitable for medicinal application to an infection prone skin area comprising: a solid hydrophobic acrylic adhesive; an antibacterial effective amount of silver sulfadiazine; and an effective amount of internally crosslinked sodium carboxymethylcellulose to release said silver sulfadiazine from said adhesive composition.

2. A composition according to claim 1 comprising by weight of the composition 40 to 50% of solid hydrophobic acrylic adhesive; 0.5 to 3.0% of silver sulfadiazine; and 1 to 10% of internally crosslinked sodium carboxymethylcellulose.

3. A composition according to claim 2 wherein the silver sulfadiazine is present at about 1% by weight of the composition.

4. A composition according to claim 2 wherein the internally crosslinked sodium carboxymethylcellulose is present at 2 to 3% by weight of the composition.

5. A composition according to claim 1 wherein the silver sulfadiazine is present at about 1% and the internally crosslinked sodium carboxymethylcellulose is present at about 2% by weight of the composition.

6. A composition according to claim 2 wherein the internally crosslinked sodium carboxymethylcellulose is a reaction product of alkali cellulose and sodium monochloroacetate.

7. The use of silver sulfadiazine in the manufacture of an antibacterial pressure sensitive adhesive composition for use in a method of reducing bacteria in an infection prone skin area, said composition comprising a mixture of: a hydrophobic adhesive; an antibacterial effective amount of silver sulfadiazine; and an amount of internally crosslinked sodium carboxymethylcellulose sufficient to make an antibacterial effective amount of the silver sulfadiazine available to the infection prone skin area to effectively reduce the bacteria present in the area.

8. The use of silver sulfadiazine as claimed in claim 7, wherein said adhesive composition is as claimed in any of claims 2 to 6.

## Patentansprüche

1. Antibakterielle, druckempfindliche Adhäsionsmasse, die zur medizinischen Anwendung auf für Infektionen anfälligen Hautbereichen geeignet ist, umfassend: ein festes, hydrophobes Acryladhäsiv, eine antibakteriell wirksame Menge Silbersulfadiazin und eine wirksame Menge intern quervernetzter Natriumcarboxymethylcellulose, um das Silbersulfadiazin aus der Adhäsivmasse freizusetzen.

2. Masse nach Anspruch 1, umfassend, bezogen auf das Gewicht der Masse, 40 - 50 % festes, hydrophobes Acryladhäsiv, 0,5 - 3,0 % Silbersulfadiazin und 1 - 10 % intern quervernetzte Natriumcarboxymethylcellulose.

3. Masse nach Anspruch 2, wobei das Silbersulfadiazin zu etwa 1 Gew.-% der Masse vorliegt.

4. Masse nach Anspruch 2, wobei die intern quervernetzte Natriumcarboxymethylcellulose zu 2 - 3 Gew.-% der Masse vorliegt.

5. Masse nach Anspruch 1, wobei das Silbersulfadiazin zu etwa 1 % vorliegt und die intern quervernetzte Natriumcarboxymethylcellulose zu etwa 2 Gew.-% der Masse vorliegt.

6. Masse nach Anspruch 2, wobei die intern quervernetzte Natriumcarboxymethylcellulose ein Reaktionsprodukt aus Alkalicellulose und Natriummonochloracetat ist.

7. Verwendung von Silbersulfadiazin bei der Herstellung einer antibakteriellen, druckempfindlichen Adhäsivmasse, die in einem Verfahren zur Reduzierung von Bakterien in einem für Infektionen anfälligen

Hautbereich verwendet wird, wobei die Masse ein Gemisch aus einem hydrophoben Adhäsiv, einer antibakteriell wirksamen Menge Silbersulfadiazin und einer Menge intern quervernetzter Natriumcarboxymethylcellulose umfaßt, die ausreicht, um eine antibakteriell wirksame Menge Silbersulfadiazin für den für Infektionen anfälligen Hautbereich verfügbar zu machen, um die in dem Bereich vorhandenen Bakterien wirksam zu reduzieren.

**8.** Verwendung von Silbersulfadiazin nach Anspruch 7, wobei die Adhäsivmasse eine Adhäsivmasse nach einem der Ansprüche 2 - 6 ist.

## Revendications

**1.** Une composition adhésive antibactérienne sensible à la pression appropriée pour une application médicale sur une zone cutanée sujette à l'infection, comprenant : un adhésif acrylique hydrophobe solide ; une quantité antibactérienne efficace de sulfadiazine d'argent ; et une quantité efficace de carboxyméthylcellulose de sodium à réticulation interne afin de libérer ledit sulfadiazine d'argent de ladite composition adhésive.

**2.** Une composition selon la revendication 1, comprenant de 40 à 50 % en masse de la composition d'adhésif acrylique hydrophobe solide ; de 0,5 à 3,0 % en masse de la composition de sulfadiazine d'argent ; et de 1 à 10 % en masse de la composition de carboxyméthylcellulose de sodium à réticulation interne.

**3.** Une composition selon la revendication 2, dans laquelle le sulfadiazine d'argent est présent à un niveau d'environ 1 % en masse de la composition.

**4.** Une composition selon la revendication 2, dans laquelle la carboxyméthylcellulose de sodium à réticulation interne est présente à un niveau allant de 2 à 3 % en masse de la composition.

**5.** Une composition selon la revendication 1, dans laquelle le sulfadiazine d'argent est présent à un niveau d'environ 1 % et la carboxyméthylcellulose de sodium à réticulation interne est présente à un niveau d'environ 2 % en masse de la composition.

**6.** Une composition selon la revendication 2, dans laquelle la carboxyméthylcellulose de sodium à réticulation interne est un produit de réaction de cellulose alcali et de monochloroacétate de sodium.

**7.** Utilisation de sulfadiazine d'argent dans la fabrication d'une composition adhésive antibactérienne sensible à la pression pouvant être utilisée dans un procédé de réduction du nombre de bactéries sur une zone cutanée sujette à l'infection, ladite composition comprenant un mélange de : un adhésif hydrophobe; une quantité antibactérienne efficace de sulfadiazine d'argent ; et une quantité suffisante de carboxyméthylcellulose de sodium à réticulation interne pour rendre disponible une quantité antibactérienne efficace de sulfadiazine d'argent sur la zone cutanée sujette à l'infection afin de réduire de façon efficace le nombre de bactéries présentes sur la zone.

**8.** L'utilisation de sulfadiazine d'argent selon la revendication 7, dans laquelle ladite composition adhésive est selon l'une des revendications 2 à 6.